(19) Europäisches Patentamt
European Patent Office
Office européen des brevets

(11) **EP 1 892 011 B1**

(12) **EUROPÄISCHE PATENTSCHRIFT**

(45) Veröffentlichungstag und Bekanntmachung des Hinweises auf die Patenterteilung:
**18.05.2016 Patentblatt 2016/20**

(51) Int Cl.:
***A61N 1/372*** *(2006.01)* ***H04B 1/16*** *(2006.01)*

(21) Anmeldenummer: **07014652.7**

(22) Anmeldetag: **26.07.2007**

(54) **Elektromedizinisches Implantat**

Electromedical implant

Implant électromédical

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **22.08.2006 DE 102006039345**

(43) Veröffentlichungstag der Anmeldung:
**27.02.2008 Patentblatt 2008/09**

(73) Patentinhaber: **Biotronik CRM Patent AG**
**6341 Baar (CH)**

(72) Erfinder:
• **Bungartz, Jörn**
**13086 Berlin (DE)**

• **Reinke, Joachim**
**10439 Berlin (DE)**

(74) Vertreter: **Galander, Marcus et al**
**Biotronik SE & Co. KG**
**Corporate Intellectual Properties**
**Woermannkehre 1**
**12359 Berlin (DE)**

(56) Entgegenhaltungen:
**EP-A- 1 353 447 WO-A-2005/062644**
**WO-A-2005/099817 US-A1- 2001 041 551**
**US-A1- 2003 119 568 US-A1- 2006 122 667**

**Beschreibung**

Die Erfindung betrifft ein implantierbares medizinisches Gerät mit einem eingebauten Sender/Empfänger (Transceiver) zum drahtlosen Senden und Empfangen von Daten, die zwischen einzelnen Datenübertragungen abgeschaltet werden kann.

Solche implantierbaren medizinischen Geräte können beispielsweise Herzschrittmacher oder Kardioverter/Defibrillatoren oder Kombinationen aus beiden sein. Mittlerweile sind eine Vielzahl solcher Implantate bekannt, die einen Sender/Empfänger, also eine Transceiver aufweisen, mit dessen Hilfe es möglich ist, physiologische oder technische Daten oder beides aus dem Implantat auf ein externes Gerät zu übertragen oder umgekehrt mit Hilfe eines externen Gerätes Daten zum Implantat zu übertragen. Letzteres kann beispielsweise zur Programmierung des Implantates oder aber auch zur Abfrage bestimmter Daten wünschenswert sein. Solche Sender/Empfänger sind z.B. aus WO 2005/099817A und US 2006/122667A1 bekannt.

Grundsätzlich stellt sich bei solchen Implantaten immer das Problem, dass die Energieressourcen des Implantats begrenzt sind und meist durch eine fest in das Implantat eingebaute Batterie gegeben sind. Es besteht daher grundsätzlich die Aufgabe, den Energieverbrauch des Implantats möglichst zu beschränken. Dies kann beispielsweise dadurch geschehen, dass momentan nicht gebrauchte Bestandteile des Implantats abgeschaltet werden. In diesem Falle stellt sich das weitere Problem, wie ein Wiedereinschalten dieser Implantatteile erfolgen soll.

Darüber hinaus ist zu berücksichtigen, dass sich gegebenenfalls mehrere Implantate in gegenseitiger Reichweite befinden oder in Reichweite eines oder mehrere externer Geräte.

Aufgabe der Erfindung ist es, ein Implantat zu schaffen, das für das zuvor genannte Szenario tauglich und dabei möglichst energiesparend ist.

Die Anmeldung betrifft ein Implantat mit den Merkmalen des Patentanspruchs 1. Erfindungsgemäß wird diese Aufgabe durch ein Implantat der eingangs genannten Art gelöst, das neben dem Transceiver eine Aufweckeinheit aufweist, die dazu ausgebildet ist, den Transceiver aus seinem Ruhezustand oder aus seinem ausgeschalteten Zustand, in dem er wenig oder keine Energie benötigt, in seinen voll betriebsbereiten Zustand zu schalten, in dem er entsprechend mehr Energie benötigt. Hierzu weist die Aufweckeinheit einen zweiten, separaten Low Power Empfänger auf, der einen wesentlich geringeren Energiebedarf hat als der Transceiver in seinem eingeschalteten bzw. voll betriebsbereiten Zustand. Außerdem weist die Aufweckeinheit eine Aufweck-Steuereinheit auf, die mit einem Ausgang des Low Power Empfängers verbunden ist. US 2003/119568A1, US 2001/041551A1, EP 1353447A und WO 2006/062644A offenbaren solche Low Power Empfänger, die eine einzelne Frequenz überwachen. Der Low Power Empfänger ist ausgebildet, eine Mehrzahl vorgegebener Frequenzbereiche derart zu überwachen, dass er im Falle einer Übertragung ausreichender Signalstärke in einem oder mehreren der Frequenzbereiche ein Ausgangssignal erzeugt und an die Aufweck-Steuereinheit abgibt. Die Aufweck-Steuereinheit ist ausgebildet, Ausgangssignale des Low Power Empfängers auszuwerten und ein Aufwecksignal an den Transceiver abzugeben, der diesen ein- oder voll betriebsbereit schaltet, falls eine vorgegebene Bedingung erfüllt ist oder eine Mehrzahl vorgegebener Bedingungen erfüllt sind. Dabei ist die Aufweck-Steuereinheit so ausgebildet, dass sie dann ein Aufwecksignal an den Transceiver abgibt, wenn der Low Power Empfänger eine Folge von Ausgangssignalen abgibt, die kennzeichnen, dass der Low Power Empfänger eine Folge von Übertragungen ausreichender Signalstärke in verschiedenen Frequenzbereichen erfasst hat, die einer vorgegebenen Abfolge oder Reihenfolge entsprechen. Wenn die von dem Low Power Empfänger überwachten Frequenzbereiche beispielsweise die Frequenzbereiche A, B, C und D sind, kann die vorgegebene Abfolge bzw. Reihenfolge derart sein, dass die Ausgangssignale Übertragungen in den Frequenzbereichen C, A, B, D (in dieser Reihenfolge) kennzeichnen.

Eine derartige Abfolge von Übertragungen in verschiedenen Frequenzbereichen soll im Folgenden auch als Triggersignalfolge bezeichnet werden, da eine derartige Folge von Übertragungen dazu dienen soll, das Einschalten des Transceivers auszulösen. Die Reihenfolge der Ausgangssignale des Low Power Empfängers kennzeichnet somit immer ein jeweiliges Frequenzschema. Ein Frequenzschema dient quasi als Schlüssel für das Einschalten des Transceivers eines entsprechend voreingestellten oder programmierten Implantats.

Ergänzend oder auch ersatzweise zu der Vorgabe einer Reihenfolge, also eines Frequenzschemas, können auch einzelne Zeiten vorgegeben sein, zu denen die Signale aufeinander folgen müssen. Bei der ersatzweisen Vorgabe der Reihenfolge kann beispielsweise die Aufweckeinheit allein die zeitliche Abfolge von Signalen auf nur einem Frequenzband überwachen.

Zum vorgenannten Zweck weist der Low Power Empfänger vorzugsweise mehrere Bandpassfilter auf, deren Durchlassbereich an die vorgegebenen Frequenzbereiche angepasst ist. Jedem Bandpassfilter ist ein Signaldetektor zugeordnet, der derart mit dem jeweiligen Bandpassfilter zusammenwirkt, dass der Signaldetektor ein Signal ausgibt, wenn der Low Power Empfänger in einem jeweiligen Frequenzbereich, der einem Durchlassbereich desjenigen Bandfilters entspricht, dem der Signaldetektor zugeordnet ist, eine Übertragung mit ausreichender Signalstärke empfängt. Bei einem derartigen Low Power Empfänger liegt am Ausgang eines jeweiligen Signaldetektors ein Ausgangssignal zur Weitergabe an die Aufweck-Steuereinheit vor, sobald der Low Power Empfänger eine Übertragung ausreichender Signalstärke im jeweiligen Frequenzbereich empfängt. Auf diese Weise erzeugt der Low Power Empfänger Signale bzw.

Signalfolgen, die von der Aufweck-Steuereinheit weiterzuverarbeiten sind.

Vorzugsweise ist die Aufweck-Steuereinheit ausgebildet, die Reihenfolge der von den Signaldetektoren ausgegebenen Signale zu erfassen und mit einer vorgegebenen Reihenfolge zu vergleichen, um im Falle eines positiven Vergleichs (die empfangene Signalfolge entspricht der vorgegebenen Reihenfolge) das Aufwecksignal an den Transceiver abzugeben.

Zusätzlich kann die Aufweck-Steuereinheit eine Zeitüberwachungseinheit aufweisen und ausgebildet sein, das Aufwecksignal nur dann zu erzeugen, wenn die von den Signaldetektoren ausgegebenen Signale innerhalb einer vorgegebenen Zeitdauer nacheinander auftreten. Die vorgegebene Zeitdauer kann dabei eine Gesamtzeitdauer sein, innerhalb der alle Signale aufgetreten sein müssen und es können auch mehrere Zeitdauern vorgegeben sein, die beschreiben, bis wann ein nachfolgendes Signal auf das jeweils vorangehende Signal folgen muss.

In allen Ausführungsvarianten ergibt sich ein implantierbares medizinisches Gerät, dessen Transceiver durch Empfangen einer Triggersignalfolge von einem ausgeschaltetem oder einem Stromsparmodus in einen eingeschalteten bzw. voll betriebsbereiten Modus geschaltet werden kann, ohne dass dazu ein Signal mit ausreichend guter Qualität empfangen werden muss, um das Signal dekodieren zu können. Daher reicht ein einfacher Empfänger als Low Power Empfänger mit geringem Energiebedarf. Dennoch ist ein gezieltes Ansprechen eines entsprechend voreingestellten Implantats möglich, auch ohne dass dafür zunächst beispielsweise entsprechende Adressdaten in einem empfangenen Signal dekodiert und ausgewertet werden müssen.

Ein weiterer Aspekt betrifft die Reaktion des Implantats auf den Empfang eines Triggersignals bzw. einer Triggersignalfolge. Wenn beispielsweise eine Mehrzahl von Implantaten durch eine Triggersignalfolge gleichzeitig angesprochen wird, könnte dies zur Folge haben, dass alle angesprochenen Implantate gleichzeitig ein Antwortsignal auf die Trägersignalfolge aussenden, so dass wenigstens für die Mehrzahl der Implantate eine erfolgreiche Kommunikation mit einem externen Gerät nicht möglich ist.

Um diesem Problem zu begegnen, weist das implantierbare medizinische Gerät vorzugsweise eine Sendesteuereinheit auf, die einen Zufallsgenerator aufweist oder mit einem solchen verbunden und ausgebildet ist, nach Ablauf einer Wartezeit nach dem Einschalten des Transceivers durch die Aufweck-Steuereinheit ein Antwortsignal auszusenden und dazu den Zeitpunkt eines Sendebeginns nach dem Einschalten des Transceivers durch die Aufweck-Steuereinheit derart zu bestimmen, dass der Zeitpunkt des Sendebeginns dem Endzeitpunkt der Wartezeit entspricht, die Ihrerseits mit dem Aufwecksignal beginnt. Die Wartezeit hat dabei eine Dauer, die dem Produkt ZZ x SD aus einer von dem Zufallsgenerator erzeugten Zufallszahl ZZ und einer vorbestimmten, durchschnittlichen Sendedauer SD entspricht.

Auf diese Weise ergibt sich, dass die Wartezeit, nach der ein Implantat auf ein entsprechendes Triggersignal reagiert, eine zufällige Länge hat, so dass es unwahrscheinlich ist, dass zwei Implantate gleichzeitig auf ein Triggersignal antworten.

Wenn darüber hinaus gemäß einer bevorzugten Ausführungsvariante die Zufallszahl derart skaliert ist, dass sie eine ganze Zahl zwischen 0 und einer Höchstzahl zu erwartender Implantate im Empfangsbereich eines externen Gerätes minus 1 ist, ist es außerdem sehr unwahrscheinlich, dass während der Sendedauer für die Übertragung des Antwortsignals eines ersten Implantats ein zweites Implantat mit dem Senden eines Antwortsignals beginnt.

Bei einer vorgegebenen, maximal zu erwartenden Sendedauer SD bestimmt diese Zeitdauer SD jeweils einen Timeslot für das Senden des Antwortsignals. Da alle Implantate in der Umgebung des externen Geräts durch das gleiche Triggersignal seitens des externen Gerätes aufgeweckt werden oder - in einer allgemeineren Ausführungsform - zumindest synchronisiert werden, sind die Timeslots, sprich die in Abhängigkeit der jeweiligen Zufallszahl ZZ hintereinander geschalteten Sendedauerzeiten SD gleichgetaktet.

In einem derartigen Szenario sind aufgrund der Nähe der Implantate zum externen Gerät Signallaufzeiten zwischen den Implantaten und dem externen Gerät vernachlässigbar.

Für ein solches Szenario ergibt sich für eine für alle Implantate gleich vorgegebene Maximalzahl von Implantaten AI, die auf jeden Fall größer ist als die tatsächliche Anzahl n der Implantate eine Wahrscheinlichkeit $P_{AI,n}$, dass alle Implantate in verschiedenen Timeslots senden als:

$$P_{AI,n} = P_{AI,n} = \frac{\prod_{i}^{AI} = AI - n + 1^{i}}{AI^{n}}.$$

Für $AI \rightarrow \infty$ wird diese Wahrscheinlichkeit $P_{AI,n}1$, d. h., dass für eine vergleichsweise groß vorgegebenen Zahl AI es sehr wahrscheinlich ist, dass die Implantate jeweils in einem eigenen Timeslot senden, also keine zwei Implantate im gleichen Timeslot senden. Dies ist unabhängig von der durch die maximale Sendezeitdauer SD vorgegebenen Länge der Timeslots.

Da die Wahrscheinlichkeit, dass zwei Implantate dennoch ein Antwortsignal im gleichen Timeslot senden, zwar nahe 0, aber nicht gleich 0 ist, ist die Sendesteuereinheit in einer bevorzugten Ausführungsvariante dazu ausgebildet, das Senden des Antwortsignals nach Ablauf einer erneut bestimmten Wartezeit zu wiederholen. Dabei kann das Wiederholen des Sendens des Antwortsignals davon abhängig sein, ob ein jeweils gesendetes Antwortsignal unbeantwortet bleibt oder nicht.

Die Erfindung soll nun anhand eines Ausführungsbeispiels mit Bezug auf die Figuren näher erläutert werden. Von den Figuren zeigen:

Figur 1: beispielhaft ein Szenario mit einem externen Gerät und zwei Implantaten in der Reichweite des externen Gerätes;

Figur 2: den Aufbau der Sende- und Empfangseinheit eines Implantates;

und Figur 3: einen Low Power Empfänger, der Sende- und Empfangseinheit des Implantates aus Figur 2.

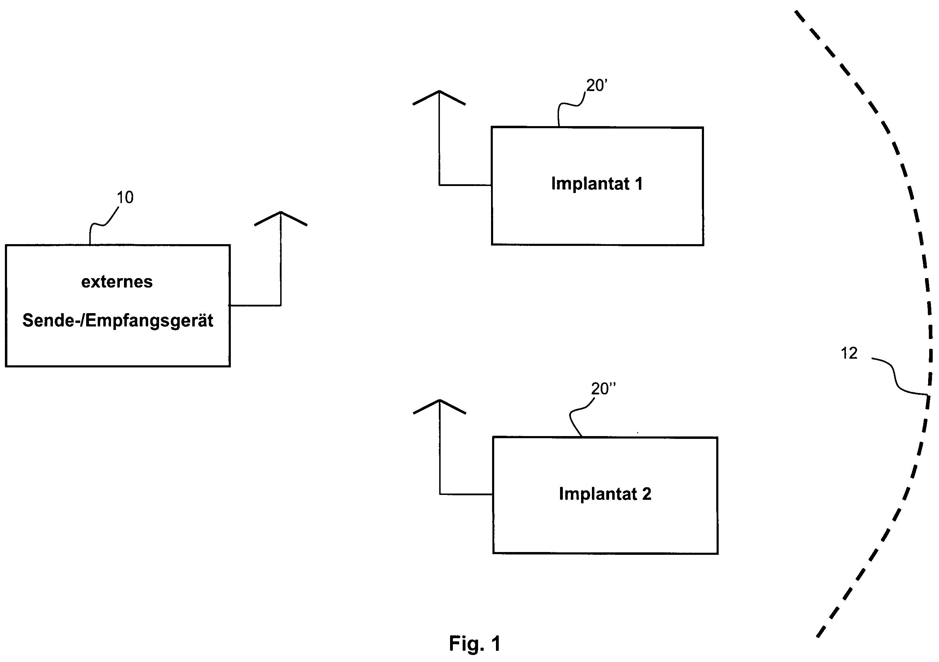

Figur 1 zeigt ein externes Gerät als externes Sende-/Empfangsgerät 10 sowie zwei Implantate 20' und 20", die sich innerhalb einer durch eine gestrichelte Linie 12 angedeuteten Empfangsreichweite des externen Gerätes 10 befinden. Die Empfangsreichweite ergibt sich aus der Sendeleistung der Implantate 20' und 20" sowie der Empfindlichkeit des Empfängers des externen Gerätes 10.

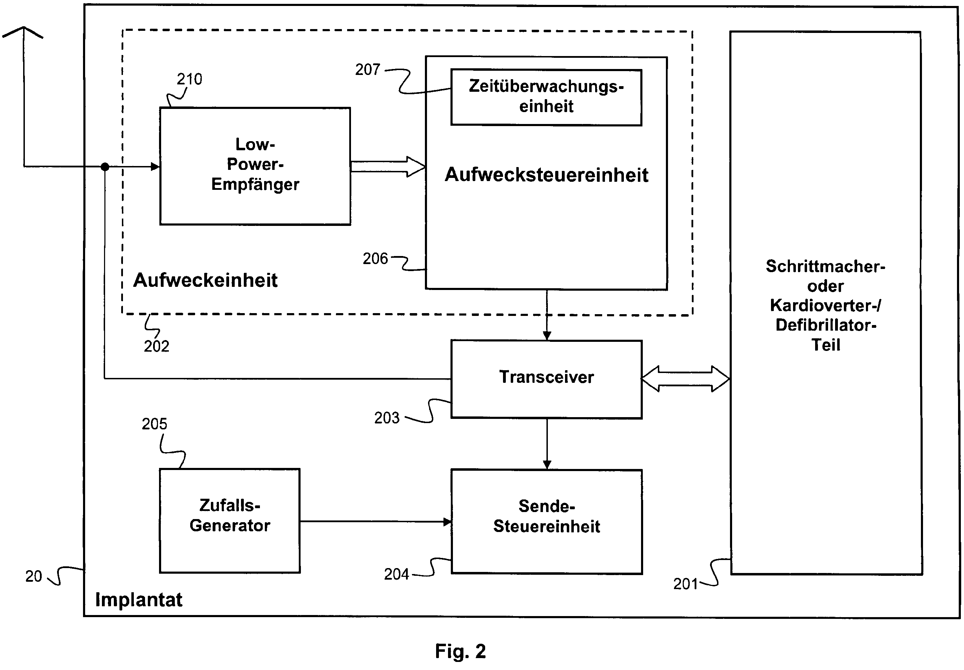

Im Zusammenhang mit dem in Figur 1 dargestellten Szenario ergibt sich zum einen grundsätzlich das Problem, dass die Implantate jeweils einen Transceiver 203 (siehe Figur 2) aufweisen, der im Sende- und Empfangsmodus relativ viel Energie verbraucht und daher möglichst lange und möglichst häufig in einem Energiesparmodus gehalten oder ausgeschaltet sein sollte, gleichzeitig aber auch durch ein Signal von außerhalb des Implantats in seinen voll betriebsbereiten Modus zu schalten sein sollte. Dies soll aber nach Möglichkeit nicht durch Signale geschehen, die von einem anderen Implantat oder völlig fremden sendenden Gerät in der Empfangsreichweite eines jeweiligen Gerätes stammen. Wenn möglich, soll der Transceiver 203 eines jeweiligen Implantates möglichst nur durch ein externes Gerät wie das externe Gerät 10 aufzuwecken sein.

Aus dem in Figur 1 dargestellten Szenario ergibt sich außerdem das Problem, dass nicht beide Implantate 20' und 20" gleichzeitig mit dem externen Gerät 10 im gleichen Frequenzbereich drahtlos kommunizieren können.

Das in Figur 2 abgebildete Implantat 20 stellt eine bevorzugte Ausführungsvariante eines Implantats dar, die beiden zuvor genannten Problemen Rechnung trägt.

Zum einen weist das Implantat 20 eine Aufweckeinheit 202 auf, die mit dem Transceiver 203 des Implantats 20 verbunden ist und an den Transceiver 203 ein Aufwecksignal ausgeben kann, durch welches der Transceiver 203 von einem ausgeschalteten Zustand oder einem Energiesparmodus in einen mehr Energie benötigenden, voll betriebsbereiten Modus geschaltet werden kann.

Das Aufwecksignal, das die Aufweckeinheit 202 an den Transceiver 203 abgibt, soll drahtlos ausgelöst werden können, aber nicht durch eine beliebige Datenübertragung. Außerdem soll der Empfang eines das Aufwecksignal auslösenden, drahtlos übertragenden Triggersignals nicht bereits soviel Energie benötigen, wie der Transceiver in 203 in seinem voll betriebsbereiten Zustand benötigt.

Um dies zu erreichen, weist die Aufweckeinheit 202 neben einer Aufwecksteuereinheit 207, die letztendlich das Aufwecksignal erzeugt, einen Low Power Empfänger 210 auf, der als breitbandiger Empfänger dazu in der Lage ist, drahtlose Übertragungen von Signalen in verschiedenen Frequenzbereichen zu erfassen. Konkret ist der Low Power Empfänger 210 dazu in der Lage, Übertragungen, die jeweils eine minimale, vorgegebene Signalstärke überschreiten, in einem von mehreren vorgegebenen Frequenzbereichen zu erfassen und jeweils ein Ausgangssignal zu erzeugen, falls er in einem der vorgegebenen Frequenzbereiche eine Übertragung mit einer Signalstärke oberhalb des vorgegebenen Minimums erfasst.

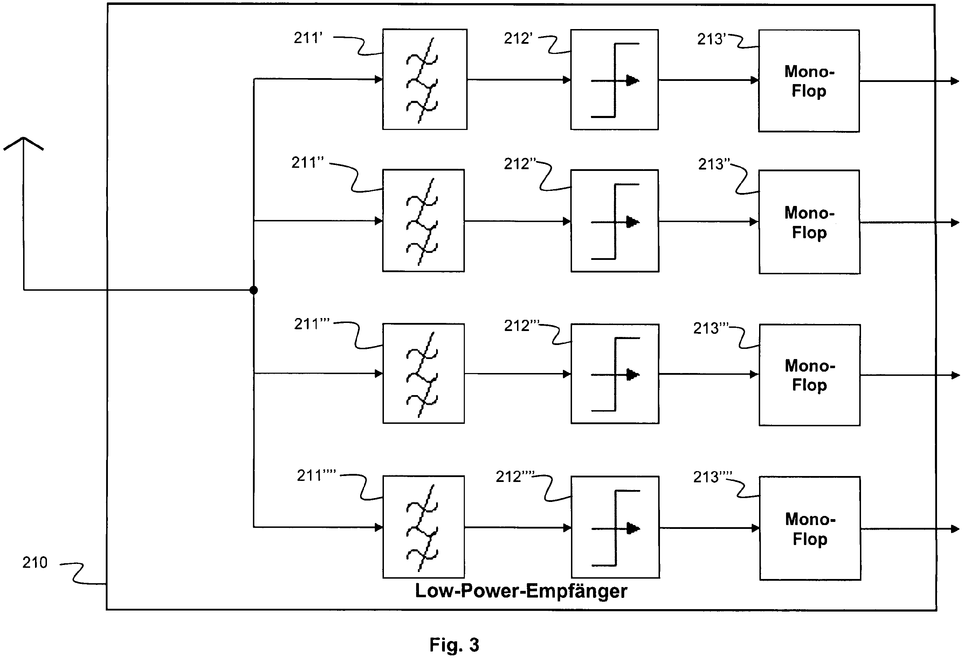

Hierzu weist der Low Power Empfänger (siehe Figur 3) vier Bandpassfilter 211', 211", 211''' und 211"" auf, die jeweils einen Durchlassbereich (Passband) haben, der jeweils auf einen Frequenzbereich von insgesamt vier Frequenzbereichen abgestimmt ist. Jedem Bandpassfilter 211', 211", 211''' und 211"" ist jeweils ein Signaldetektor 212', 213', 212", 213", 212''', 213'', 212"", 213"" nachgeschaltet. Jeder dieser Signaldetektoren weist einen Schwellwertschalter 212', 212", 212''' und 212"" auf, der darauf anspricht, wenn am Ausgang des entsprechend zugeordneten Bandpassfilters 211', 211", 211''' oder 211"" ein Ausgangssignal an einer Signalstärke anliegt, die oberhalb der vorgegebenen Schwelle liegt. Wenn der jeweilige Schwellwertschalter 212', 212", 212''' oder 212"" entsprechend auf ein derartiges Signal anspricht, löst er eine jeweilige, dem Schwellwertschalter 212', 212", 212''' oder 212"" nachgeschaltete monostabile Kippstufe (Monoflop) 213', 213", 213''' oder 213"" aus. Die jeweilige monostabile Kippstufe 213', 213", 213''' oder 213"" erzeugt auf diese Weise ein Ausgangssignal, welches kennzeichnet, dass in dem über dem Durchlassbereich des jeweiligen Bandpassfilters 211', 211", 211''' oder 211"" vorgegebenen Frequenzbereich eine Übertragung von Signalen mit einer Signalstärke oberhalb des vorgegebenen Minimum stattgefunden hat. Auf diese Weise kennzeichnen die Ausgangssignale der monostabilen Kippstufen 213', 213", 213''' und 213"", die gleichzeitig Ausgangssignale des Low Power Empfängers 210 sind, durch ihre Reihenfolge die Reihenfolge, mit der Übertragungen in verschiedenen Frequenzbereichen seitens des Low Power Empfängers 210 empfangen werden.

Die Aufweck-Steuereinheit 206 ist ausgebildet, die Ausgangssignale des Low Power Empfängers 210 in zweierlei Hinsicht auszuwerten. Zum einen ist die Aufweck-Steuereinheit dazu ausgebildet, die Reihenfolge der Ausgangssignale des Low Power Empfängers 210 mit einer vorgegebenen Reihenfolge zu vergleichen und nur dann das Aufwecksignal an den Transceiver 203 auszugeben, wenn die Reihenfolge der Ausgangssignale des Low Power Empfängers 210 der vorgegebenen, im Implantat 20 gespeicherten Reihenfolge entspricht. Zusätzlich ist die Aufweck-Steuereinheit 206 dazu ausgebildet, mit Hilfe einer Zeitüberwachungseinheit 207 sicherzustellen, dass das Aufweck-Signal nur dann erzeugt wird, wenn die Ausgangssignale des Low Power Empfängers 210 nicht nur in der vorgegebenen Reihenfolge, sondern auch innerhalb jeweils vorgegebener Zeit aufeinander folgend eintreffen.

Die vorgegebene Reihenfolge der Signale sowie die entsprechend vorgegebenen Zeiten ergeben eine Art charakteristischen Schlüssel, mit dem beispielsweise ein externes Gerät auf drahtlosem Wege einen Transceiver 203 eines Implantats 20 gezielt aufwecken kann, ohne dass dazu Daten dekodiert und ausgewertet werden müssen, die beispielsweise als Adressdaten in einem drahtlos übertragenen Signal enthalten sein könnten.

Eine Sendesteuereinheit 204 des Implantats 20 ist dazu ausgebildet, das Aussenden eines Antwortsignals zu steuern, welches das Implantat 20 nach Empfang eines drahtlos übertragenen Triggersignals, welches über ein entsprechendes Aufwecksignal zum Aufwecken des Transceivers 203 geführt hat, auszusenden. Die Sendesteuereinheit ist dazu ausgebildet, ein Aussenden des Antwortsignals über den Transceiver 203 erst nach Ablauf einer Wartezeit auszulösen, die mit dem Aufwecken des Transceivers 203 beginnt. Die Sendesteuereinheit 204 berechnet diese Wartezeit aus einer von einem Zufallsgenerator 205 erzeugten Zufallszahl ZZ und einem vorgegebenen Maximalwert für eine Sendedauer SD für das Senden des Antwortsignals B aus einer ebenfalls vorgegebenen ganzen Zahl AI, die größer ist, als die maximal zu erwartende Anzahl von Implantaten innerhalb der Reichweite eines externen Gerätes.

Aus der auf ganze Zahlen zwischen 0 und AI-1 skalierten Zufallszahl ZZ, und der vorgegebenen, im Implantat gespeicherten maximalen Sendedauer SD bildet die Wartezeit als Produkt aus der ganzzahligen, mit AI skalierten Zufallszahl ZZ und SD: ZZ x SD.

Die jeweilige, so bestimmte Wartezeit wird mit Empfang des Trägersignals gestartet und führt dazu, dass die Steuereinheit 204 das Aussenden des Antwortsignals über den Receiver 203 zum Ende der Wartezeit auslöst.

Wie zuvor bereits erläutert, ist es äußerst unwahrscheinlich, dass mehr als ein Implantat in der Reichweite eines externen Gerätes gleichzeitig auf ein von dem externen Gerät ausgesandtes Triggersignal antwortet, wenn SD größer ist als die Sendezeit für das Senden des Antwortsignals und wenn AI größer ist als die maximale Anzahl von Implantaten, die sich innerhalb der Reichweite (in Figur 1 angedeutet durch die gestrichelte Linie 12) des externen Gerätes 10 befinden.

Damit wird wirksam vermieden, dass zwei Implantate gleichzeitig auf ein Triggersignal antworten.

## Patentansprüche

1. Implantierbares medizinisches Gerät (20) mit einem Transceiver (203) zum Senden und Empfangen von drahtlos übertragenen Daten, der zwischen einzelnen Datenübertragungen über den Transceiver (203) abgeschaltet oder in einen energiesparenden Ruhezustand geschaltet ist, sowie mit einer Aufweckeinheit (202), die ausgebildet ist, den Transceiver (203) durch ein Aufwecksignal von seinem abgeschaltetem Zustand oder seinem Ruhezustand in seinen voll betriebsbereiten Zustand zu schalten,
wobei die Aufweckeinheit einen Low-Power-Empfänger (210) und eine Aufweck-Steuereinheit (206) aufweist, von denen der Low-Power-Empfänger (210) ausgebildet ist, eine Mehrzahl vorgegebener Frequenzbereiche derart zu überwachen, dass er im Falle einer Übertragung ausreichender Signalstärke in einem oder mehreren der Frequenzbereiche ein Ausgangssignal erzeugt und an die Aufweck-Steuereinheit (206) abgibt, und von denen die Aufweck-Steuereinheit (206) ausgebildet ist, Ausgangssignale des Low-Power-Empfängers (210) auszuwerten und ein Aufwecksignal an den Transceiver (203) abzugeben, der diesen ein- oder voll betriebsbereit schaltet,
wobei die Aufweck-Steuereinheit (206) ausgebildet ist,das Aufwecksignal an den Transceiver (203) abzugeben, wenn die Bedingung erfüllt ist, dass der Low-Power-Empfänger (210) eine Folge von Ausgangssignalen abgibt, die kennzeichnen, dass der Low-Power-Empfänger (210) eine Folge von Übertragungen ausreichender Signalstärke in verschiedenen vorgegebenen Frequenzbereichen erfasst hat, die einer vorgegebenen Abfolge oder Reihenfolge von Frequenzen entspricht, **dadurch gekennzeichnet, dass** der Low-Power-Empfänger (210) mehrere Bandpassfilter (211', 211", 211''', 211'''') mit jeweils an die vorgegebenen Frequenzbereiche angepasstem Durchlassbereich und mit jeweils zugeordnetem Signaldetektor (212', 212", 212''', 212'''') aufweist, die derart zusammenwirken, dass ein jeweiliger Signaldetektor (212', 212", 212''', 212'''') ein Ausgangssignal ausgibt, wenn der Low-Power-Empfänger (210) in einem jeweiligen Frequenzbereich, der einem Durchlassbereich desjenigen Bandpassfilters (211', 211", 211''', 211'''') entspricht, dem der jeweilige Signaldetektor (212', 212", 212''', 212'''') zugeordnet ist, eine Übertragung mit ausreichender Signalstärke empfängt.

2. Implantierbares medizinisches Gerät (20) nach Anspruch 1, **dadurch gekennzeichnet, dass** die Aufwecksteuereinheit (206) ausgebildet ist,

- die Reihenfolge der von den Signaldetektoren (212', 212", 212''', 212'''') ausgegebenen Signale zu erfassen und

- mit einer vorgegebenen Reihenfolge zu vergleichen und

- im Falle eines positiven Vergleichs das Aufwecksignal an den Transceiver (203) abzugeben.

**3.** Implantierbares medizinisches Gerät (20) nach Anspruch 2, **dadurch gekennzeichnet, dass** die Aufwecksteuereinheit (206) eine Zeitüberwachungseinheit (207) aufweist und ausgebildet ist, das Aufwecksignal nur dann zu erzeugen, wenn die von den Signaldetektoren (212', 212", 212"', 212"") ausgegebenen Signale innerhalb einer vorgegebenen Zeitdauer nacheinander auftreten.

**4.** Implantierbares medizinisches Gerät (20) gemäß des Anspruchs 1, mit einer Sendesteuereinheit (204), **dadurch gekennzeichnet, dass** die Sendesteuereinheit (204) einen Zufallsgenerator (205) aufweist oder mit einem solchen verbunden und ausgebildet ist, nach Ablauf einer Wartezeit nach dem Einschalten des Transceivers (203) durch die Aufweck-Steuereinheit (206) ein Antwortsignal auszusenden und dazu den Zeitpunkt eines Sendebeginns nach Einschalten des Transceivers (203) durch die Aufweck-Steuereinheit (206) derart zu bestimmen, dass der Zeitpunkt des Sendebeginns dem Endzeitpunkt der Wartezeit entspricht, die mit dem Aufwecksignal beginnt und eine Dauer hat, die dem Produkt ZZ•SD aus einer von dem Zufallsgenerator (205) erzeugten Zufallszahl ZZ und mindestens einer vorbestimmten durchschnittlichen Sendedauer SD entspricht.

**5.** Implantierbares medizinisches Gerät (20) nach Anspruch 4, **dadurch gekennzeichnet, dass** die Sendesteuereinheit (204) ausgebildet ist, die Zufallszahl ZZ derart zu skalieren, dass die Zufallszahl ZZ eine ganze Zahl zwischen 0 und einer vorgegebenen Maximalzahl im Empfangsbereich eines externen Gerätes (10) befindlicher implantierbarer medizinischer Geräte (20', 20") minus 1 ist.

**6.** Implantierbares medizinisches Gerät (20) nach Anspruch 4 oder 5, **dadurch gekennzeichnet, dass** die Sendesteuereinheit (204) ausgebildet ist, das Senden des Antwortsignals nach Ablauf einer erneut bestimmten Wartezeit zu wiederholen.

**7.** Implantierbares medizinisches Gerät (20) nach einem der Ansprüche 1 bis 6, **dadurch gekennzeichnet, dass** das implantierbare medizinische Gerät (20) ein Herzschrittmacher oder ein Kardioverter/Defibrillator oder eine Kombination aus beidem ist.

**Claims**

**1.** An implantable medical device (20) comprising a transceiver (203) for transmitting and receiving wirelessly transmitted data, which transceiver is switched off or switched into an energy-saving rest state between individual data transmissions via said transceiver (203), said transceiver also comprising a waking unit (202), which is configured to switch said transceiver (203) from its switched-off state or its rest state into its fully operational state by a waking signal,

wherein said waking unit comprises a low-power receiver (210) and a waking control unit (206), of which said low-power receiver (210) is configured to monitor a plurality of predefined frequency ranges in such a way that, in case of a transmission of sufficient signal strength in one or more frequency ranges, said low-power receiver generates an output signal and outputs said output signal to said waking control unit (206), and of which said waking control unit (206) is configured to analyse output signals of said low-power receiver (210) and to output said waking signal to said transceiver (203), which switches said transceiver on or to fully operational,

wherein said waking control unit (206) is configured to output said waking signal to said transceiver (203) if the condition is met that said low-power receiver (210) outputs a sequence of output signals which identify that said low-power receiver (210) has detected a series of transmissions of sufficient signal strength in various predefined frequency ranges corresponding to a predefined series or sequence of frequencies,

**characterised in that** said low-power receiver (210) has multiple bandpass filters (211', 211", 211"', 211 ""), each having a transmission range adapted to the predefined frequency ranges and each having an assigned signal detector (212', 212", 212"', 212""), which work together in such a way that a particular signal detector (212', 212", 212"', 212"") outputs an output signal if said low-power receiver (210) receives a transmission having sufficient signal strength in a particular frequency range which corresponds to a transmission range of the bandpass filter (211', 211", 211"', 211"") which is assigned to said particular signal detector (212', 212", 212"', 212"").

**2.** The implantable medical device (20) according to Claim 1, **characterised in that** said waking control unit (206) is configured

- to detect the sequence of signals output by the signal detectors (212', 212", 212"', 212"") and
- to compare said sequence of signals to a predefined sequence and
- to output said waking signal to said transceiver (203) in case of a positive comparison.

3. The implantable medical device (20) according to Claim 2, **characterised in that** said waking control unit (206) has a time monitoring unit (207) and is configured to generate said waking signal only if the signals output by said signal detectors (212', 212", 212"', 212"") occur in sequence within a predefined duration.

4. The implantable medical device (20) according to Claim 1, having a transmission control unit (204), **characterised in that** said transmission control unit (204) has or is connected to a random generator (205) and is configured, after lapse of a waiting time after said transceiver (203) is turned on by said waking control unit (206), to transmit a response signal and for this purpose to define a time of a transmission beginning after said transceiver (203) is turned on by said waking control unit (206) in such a way that said time of said transmission beginning corresponds to an ending time of said waiting time, which begins with said waking signal and has a duration which corresponds to a product ZZ•SD of a random number ZZ generated by said random generator (205) and at least one predetermined average transmission duration SD.

5. The implantable medical device (20) according to Claim 4, **characterised in that** said transmission control unit (204) is configured to scale said random number ZZ in such a way that said random number ZZ is an integer between 0 and a predefined maximum number of implantable medical devices (20', 20") located in the reception range of an external device (10) minus 1.

6. The implantable medical device (20) according to Claim 4 or 5, **characterised in that** said transmission control unit (204) is configured to repeat the transmission of said response signal after lapse of a newly determined waiting time.

7. The implantable medical device (20) according to one of Claims 1 to 6, **characterised in that** said implantable medical device (20) is a cardiac pacemaker or a cardioverter/defibrillator or a combination of both.

**Revendications**

1. Appareil médical implantable (20) avec un émetteur-récepteur (203), pour l'envoi et la réception de données transmises sans fil, qui, entre des transmissions de données individuelles, est à l'arrêt ou dans une position de veille économe en énergie, ainsi qu'avec

une unité de redémarrage (202), qui est configurée pour passer l'émetteur-récepteur (203) de son état d'arrêt ou de son état de veille dans son état de fonctionnement complet par un signal de redémarrage, où l'unité de redémarrage présente un récepteur à consommation réduite (210) et une unité de commande de redémarrage (206), parmi lesquels, le récepteur à consommation réduite (210) est conçu pour contrôler une multitude de domaines de fréquences prédéfinies de telle sorte que, dans le cas d'une transmission de puissance de signal suffisante dans un ou plusieurs domaines de fréquences, il génère un signal de sortie et le transmet à l'unité de commande de démarrage (206), et parmi lesquels l'unité de commande de démarrage (206) est conçue pour évaluer des signaux de sortie du récepteur à consommation réduite (210) et délivrer un signal de redémarrage à l'émetteur-récepteur (203) qui met celui-ci en marche ou le remet en totale position de fonctionnement,
où l'unité de commande de redémarrage (206) est configurée pour délivrer le signal de redémarrage à l'émetteur-récepteur (203) lorsque la condition est remplie que le récepteur à consommation réduite (210) émet une suite de signaux de sortie qui montrent que le récepteur à consommation réduite (210) a détecté une suite de transmissions de puissance de signal suffisante dans divers domaines de fréquences prédéfinis, qui correspond à une succession ou une série prédéfinies de fréquences, **caractérisé en ce que**
le récepteur à consommation réduite (210) présente plusieurs filtres de bande passante (211', 211", 211"', 211"") avec chaque fois un domaine de passage adapté aux domaines de fréquences prédéfinis et avec chaque fois un détecteur de signaux (212', 212", 212"', 212"") associé qui agissent conjointement de telle manière qu'un détecteur de signaux (212', 212", 212"', 212"") donné émet un signal de sortie lorsque le récepteur à consommation réduite (210) reçoit une transmission avec une puissance de signal suffisante dans un domaine de fréquences qui correspond à un domaine de passage du filtre de bande passante (211', 211", 211"', 211"") auquel le détecteur de signaux (212', 212", 212"', 212"") donné est associé.

2. Appareil médical implantable (20) selon la revendication 1, **caractérisé en ce que** l'unité de commande de redémarrage (206) est configurée pour

- détecter la série de signaux émis par les détecteurs de signaux (212', 212", 212"', 212"") et
- la comparer avec une série prédéfinie et
- transmettre le signal de redémarrage à l'émetteur-récepteur (203) dans le cas d'une comparaison positive.

**3.** Appareil médical implantable (20) selon la revendication 2, **caractérisé en ce que** l'unité de commande de redémarrage (206) présente une unité de surveillance du temps (207) et est configurée pour générer le signal de redémarrage uniquement lorsque les signaux délivrés par les détecteurs de signaux (212', 212", 212"', 212"") se produisent l'un après l'autre pendant un laps de temps prédéterminé.

**4.** Appareil médical implantable (20) selon la revendication 1, avec une unité de commande d'émetteur (204), **caractérisé en ce que** l'unité de commande d'émetteur (204) présente un générateur aléatoire (205) ou est reliée avec un tel dispositif et est configurée pour émettre un signal de réponse après l'écoulement d'un temps d'attente après la mise en marche de l'émetteur-récepteur (203) par l'unité de commande de redémarrage (206) et pour déterminer pour cela le moment d'un début d'émission après la mise en marche de l'émetteur-récepteur (203) par l'unité de commande de redémarrage (206) de telle manière que le moment du début de l'émission correspond au moment de la fin du temps d'attente qui commence avec le signal de redémarrage et a une durée qui correspond au produit ZZ · SD d'un nombre aléatoire ZZ généré par le générateur aléatoire (205) et d'au moins une durée d'émission SD moyenne prédéterminée.

**5.** Appareil médical implantable (20) selon la revendication 4, **caractérisé en ce que** l'unité de commande d'émetteur (204) est configurée pour étalonner le nombre aléatoire ZZ de telle sorte que le nombre aléatoire est un nombre entier entre 0 et un nombre maximal prédéterminé d'appareils médicaux implantables (20', 20") moins 1 se situant dans le domaine de réception d'un appareil (10) externe.

**6.** Appareil médical implantable (20) selon les revendications 4 ou 5, **caractérisé en ce que** l'unité de commande d'émetteur (204) est configurée pour répéter l'émission du signal de réponse après l'écoulement d'un temps de réponse nouvellement déterminé.

**7.** Appareil médical implantable (20) selon l'une des revendications 1 à 6, **caractérisé en ce que** l'appareil médical implantable (20) est un stimulateur cardiaque ou un défibrillateur/cardioverteur ou une combinaison des deux.

Fig. 1

**Fig. 2**

EP 1 892 011 B1

**Fig. 3**

EP 1 892 011 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005099817 A
- US 2006122667 A1
- US 2003119568 A1
- US 2001041551 A1
- EP 1353447 A
- WO 2006062644 A